# EUROPÄISCHE PATENTSCHRIFT

(11) **EP 3 599 928 B1**
(45) Veröffentlichungstag und Bekanntmachung des Hinweises auf die Patenterteilung: **01.07.2020**
(21) Anmeldenummer: 17728441.1
(22) Anmeldetag: 09.05.2017
(51) Int. Cl.: A44B 11/06, A61F 5/02

(54) **GURTSCHNALLE FÜR EINEN BECKENGURT**
BELT BUCKLE FOR A LAP BELT
BOUCLE DE CEINTURE POUR UNE CEINTURE ABDOMINALE

(43) Veröffentlichungstag der Anmeldung: 05.02.2020
(73) Patentinhaber: Kartsana Medical GmbH, 73117 Wangen (DE)
(72) Erfinder: HEHENWARTER, Tobias, 80639 München (DE); HECKELSMÜLLER, Stefan, 80331 München (DE)
(74) Vertreter: Jakelski & Althoff Patentanwälte PartG mbB
(86) Internationale Anmeldenummer: PCT/DE2017/100395
(87) Internationale Veröffentlichungsnummer: WO 2018/206020

(56) Entgegenhaltungen:
- WO-A1-2014/089243
- WO-A1-2014/154947
- US-A- 3 686 715
- US-A1- 2013 324 898

## Beschreibung

Die vorliegende Erfindung betrifft eine Gurtschnalle für einen Beckengurt. Weiterhin betrifft die vorliegende Erfindung einen Beckengurt, der die Gutischnalle aufweist. Dieser ist zur Versorgung eines Beckenbruchs geeigent

### Stand der Technik

Unfallbedingte Verletzungen des Beckens bedürfen einer frühen Erkennung während der Erstversorgung am Unfallort, da eine Instabilität des Beckens großen Einfluss auf die Sterblichkeit des Betroffenen als Folge der Traumatisierung hat. Bei stark verletzten Traumapatienten kann ein hämorrhagischer Schock, der beispielsweise durch Reißen des sakralen Venenplexus, Blutung aus der Bruchstelle oder Arterienverletzungen hervorgerufen wird, schnell zum Tode führen. Derartig verursachte Blutungen können durch eine rechtzeitige Stabilisierung des Beckens unter Kontrolle gebracht werden. Dabei wird ein Gurt verwendet, der so früh als möglich um das Becken anzulegen ist.

Ein derartiger Gurt wird in seiner einfachsten Ausführungsform von einem Sanitäter so straff wie möglich zugezogen und dann in der zugezogenen Position fixiert. Hierbei ist es allerdings insbesondere bei Patienten mit deutlich unterschiedlichem Hüftumfang und unterschiedlicher Beckenanatomie oftmals schwer abzuschätzen, wann eine ausreichend hohe Zugkraft auf den Gurt angewandt wurde. Wenn die benötigte Zugkraft deutlich überschritten wird, kann der dadurch erzeugte Druck auf den gebrochenen Beckenknochen statt zu einer Stabilisierung zu einer weiteren Verschlimmerung der vorhandenen Verletzung führen.

Es wurden deshalb Gurtschnallen vorgeschlagen, welche ein weiteres Zuziehen des Gurtes bei Erreichen einer vorgegebenen Zugkraft verhindern. Derartige Gurtschnallen sind beispielsweise aus der WO03/075743 A2, der WO2011/016824 A2 und der WO2014/089243 A1 bekannt. Allen diesen Gurtschnallen ist gemeinsam, dass sie einen Gurtführungsbereich aufweisen, in welchem sich der Gurt beim Anziehen zunächst freibewegen kann. Durch die Zugkraft wird Druck auf in der Gurtschnalle angeordneten Federn ausgeübt. Hierdurch wird ein Schlitten, welcher durch die Federn in einer Ausgangsposition gehalten wird, allmählich aus dieser Ausgangsposition herausbewegt. Der Schlitten weist Öffnungen auf, in denen Stifte geführt sind. In der Ausgangsposition sind die Stifte vollständig im Schlitten versenkt. Mit zunehmender Zugkraft gleitet der Schlitten so zurück, dass die Stifte aus diesem hinausgleiten und nun in den Gurtführungsbereich hineinstehen. Diese Gurtschnallen sind für die Verwendung mit einem Beckengurt vorgesehen, welcher in regelmäßigen Abständen Löcher aufweist. Sobald sich die Stifte in den Gurtführungsbereich hineinbewegt haben, greifen diese in das nächste Lochpaar ein, das über sie hinwegbewegt wird und verhindern so ein weiteres Zuziehen des Gurtes. Auf diese Weise wird der Gurt in seiner so erreichten Position fixiert.

Dieses System ermöglicht es allerdings nicht, eine exakte Zugkraft einzustellen, bei der eine Weiterbewegung des Gurtes gestoppt werden soll. Wenn eine über die Auswahl der Federn vorgegebene Zugkraft erreicht wurde, bewegt sich der Gurt stets noch ein wenig weiter bis das nächste Lochpaar erreicht wird. Auf diese Weise wird eine Zugkraft erreicht, die über die Vorgabe hinausgeht. Es ist auch nicht möglich, den angezogenen Gurt schnell und in einfacher Weise zu lockern, wenn dies beispielsweise bei einer weiteren Versorgung des Patienten im Krankenhaus notwendig werden sollte. Schließlich können derartige Gurtschnallen konstruktionsbedingt nur mit gelochten Gurten verwendet werden.

Es ist eine Aufgabe der vorliegenden Erfindung, eine Gurtschnalle für einen Beckengurt bereitzustellen, die eine exakte Einstellung einer maximal zu erreichenden Zugkraft ermöglicht. Eine weitere Aufgabe besteht darin, die Gurtschnalle so auszuführen, dass sie auch mit einem nicht gelochten Gurt verwendet werden kann. Schließlich besteht eine Aufgabe der Erfindung darin, einen Beckengurt zur Versorgung eines Beckenbruchs bereitzustellen, welcher diese Gurtschnalle aufweist.

### Offenbarung der Erfindung

Diese Aufgabe wird in einem Aspekt der Erfindung durch eine Gurtschnalle für einen Beckengurt zur Versorgung eines Beckenbruchs gelöst, die ein Gehäuse mit einem ersten Ende und einem zweiten Ende aufweist. Ein Schlitten ist in dem Gehäuse zwischen dem ersten Ende und dem zweiten Ende beweglich angeordnet. Mindestens ein Federelement ist eingerichtet, um den Schlitten in Richtung des zweiten Endes zu drücken. Mindestens ein Stift ist so an dem Schlitten angeordnet, dass er in Richtung des zweiten Endes weist. Zwei Klemmbacken sind an dem zweiten Ende des Gehäuses drehbar angeordnet. Sie weisen Schenkelfederelemente auf, welche sie in eine geschlossene Position drücken. Ein Gurtführungsbereich verläuft zwischen dem Schlitten und den Klemmbacken.

Unter einem Federelement wird erfindungsgemäß ein Element verstanden, welches aus einer Ruheposition heraus elastisch komprimiert werden kann. Beispielsweise kann es sich hierbei um eine Spiralfeder handeln. Wenn sich das mindestens eine Federelement in einer Ruheposition befindet, drückt der mindestens eine Stift die Klemmbacken in eine geöffnete Position. Er wirkt also der Federkraft der Schenkelfederelemente entgegen. Wenn sich das mindestens eine Federelement in einer komprimierten Position befindet, drückt der mindestens eine Stift die Klemmbacken nicht in die geöffnete Position. Sie werden von den Schenkelfederelementen in die geschlossene Position gedrückt.

In der geöffneten Position der Klemmbacken kann ein Beckengurt im Gurtführungsbereich frei über den Schlitten bewegt werden. Je höher die Zugkraft beim Anziehen des Beckengurtes wird, desto stärker wird dabei das mindestens eine Federelement komprimiert. Schließlich wird der mindestens eine Stift dabei so weit von den Klemmbacken fortbewegt, dass er ein Schließen der Klemmbacken nicht mehr verhindern kann. Durch das Schließen der Klemmbacken wird der Gurt nun zwischen dem Schlitten und den Klemmbacken eingeklemmt, so dass er nicht mehr weiter angezogen werden kann. Die zu diesem Zeitpunkt erreichte Zugkraft wird also gehalten.

Die Zugkraft bei der ein Schließen der Klemmbacken erfolgt, kann durch geeignete Wahl des Federelements eingestellt werden. Dies ist vorzugsweise so ausgeführt, dass die Klemmbacken in die geschlossene Position gedrückt werden, wenn auf das mindestens eine Federelement eine vorgegebene Kompressionskraft wirkt, die im Bereich von 120 N bis 180 N liegt. Besonders bevorzugt wird für die vorgegebene Kompressionskraft ein Wert von 140 N gewählt Im Gegensatz zu herkömmlichen Gurtschnallen, welche nach Erreichen einer über ein Federelement vorgegebenen Zugkraft noch ein weiteres Anziehen des Beckengurtes erfordern bis dieser einrastet, ermöglicht es der erfindungsgemäße Beckengurt, das Anziehen exakt bei der gewünschten Kraft zu beenden.

Der Schlitten weist vorzugsweise zwei Stifte auf. Diese sind so angeordnet, dass der Gurtführungsbereich zwischen den beiden Stiften verläuft. Er wird dann also an drei Seiten von den beiden Stiften und dem Schlitten begrenzt und bei Schließen der Klemmbacken auf seiner vierten Seite von diesen geschlossen. Neben dem Verhindern eines Schließens der Klemmbacken beteiligen die Stifte sich in dieser Ausführungsform der Gurtschnalle also auch noch an der Führung des Gurtes.

Weiterhin ist es bevorzugt, dass der Schlitten einen Hohlraum aufweist. In diesem ist das mindestens ein Federelement angeordnet. Dies ermöglicht eine kompakte Ausführung der Gurtschnalle, wobei das Federelement gleichzeitig vor Umwelteinflüssen geschützt wird.

Insbesondere wenn das mindestens eine Federelement als Spiralfeder ausgeführt ist, ist es bevorzugt, dass die Gurtschnalle mehrere Federelemente, insbesondere zwei Federelemente aufweist, um eine gleichmäßige Kraftaufnahme zu ermöglichen.

Der Schlitten weist auf seiner den Klemmbacken zugewandten Seite vorzugsweise eine Außenkontur auf, welche im Wesentlichen komplementär zur Außenkontur der geschlossenen Klemmbacken auf ihrer dem Schlitten zugewandten Seite ist. Bei geschlossenen Klemmbacken bewirkt dies einen maximalen Klemmbereich des Gurtes zwischen dem Schlitten und den Klemmbacken. Hierdurch wird einerseits sichergestellt, dass auch durch große Kraftanwendung kein weiteres Zuziehen des Gurtes mehr möglich ist. Andererseits wird auch ein ungewolltes Lockern des Gurtes verhindert. Die Außenkontur des Schlittens ist dabei insbesondere konvex. Unter konvex wird hierbei sowohl eine gerundet konvexe Form als auch eine polygonal konvexe Form, wie insbesondere eine dreieckig konvexe Form verstanden. Die komplementäre Außenkontur der geschlossenen Klemmbacken ist dann demgemäß konkav, wobei auch unter konkav sowohl eine gerundet konkave als auch eine polygonal konkave, insbesondere eine dreieckig konkave Kontur verstanden wird.

Um ein symmetrisches Öffnen und Schließen der Klemmbacken zu gewährleisten, ist es bevorzugt, dass diese miteinander verzahnt sind.

Mindestens eine der Klemmbacken weist vorzugsweise ein von außerhalb des Gehäuses zugängliches Bedienelement auf. Dieses ist eingerichtet, um die Klemmbacke aus einer geschlossenen in eine geöffnete Position zu bewegen. Dies ermöglicht in einfacher Weise ein Lockern des Beckengurtes. Wird auf diesen keine Zugkraft angewandt, während das Bedienelement betätigt wird, so entspannt er sich und das mindestens eine Federelement kehrt in seine Ruheposition zurück. Damit werden die Klemmbacken mittels des mindestens einen Stiftes in ihre geöffnete Position zurückgebracht. Wenn die Klemmbacken miteinander verzahnt sind, kann durch Betätigen eines Bedieneiements an nur einer Klemmbacke auch die andere Klemmbacke gleichzeitig geöffnet werden.

Es ist weiterhin bevorzugt, dass die Gurtschnalle am zweiten Ende des Gehäuses ein Gurtbefestigungselement aufweist. Hierbei handelt es sich insbesondere um eine Steckschnalle. Das Gurtbefestigungselement ermöglicht es, einen Beckengurt zunächst locker um das Becken eines Patienten herum zu legen, bevor dieser dann angezogen wird.

in einem weiteren Aspekt betrifft die Erfindung einen Beckengurt zur Versorgung eines Beckenbruchs. Dieser ist an seinem einen Ende an dem Gurtbefestigungselement der Gurtschnalle befestigt. Mit seinem anderen Ende ist er durch den Gurtführungsbereich der Gurtschnalle geführt. Er kann im Gurtführungsbereich so lange angezogen werden bis eine durch die Gurtschnalle vorgegebene Zugkraft erreicht wurde und er durch Schließen der Klemmbacken fixiert wird.

### Kurze Beschreibung der Zeichnungen

Ein Ausführungsbeispiel der Erfindung ist in den Zeichnungen dargestellt und wird in der nachfolgenden Beschreibung näher erläutert.
- Fig. 1: zeigt eine isometrische Darstellung eines Beckengurtes mit einer Gurtschnalle gemäß einem Ausführungsbeispiel der Erfindung.
- Fig. 2: zeigt eine Explosionsdarstellung eines Beckengurtes mit einer Gurtschnalle gemäß einem Ausführungsbeispiel der Erfindung.
- Fig. 3: zeigt eine seitliche Schnittdarstellung eines Beckengurtes mit einer Gurtschnalle gemäß einem Ausführungsbeispiel der Erfindung, wobei die Klemmbacken sich in ihrer geöffneten Position befinden.
- Fig. 4: zeigt in einer seitlichen Schnittdarstellung einen Beckengurt mit einer Gurtschnalle gemäß einem Ausführungsbeispiel der Erfindung, wobei die Klemmbacken sich in einer geschlossenen Position befinden.

### Ausführungsbeispiele der Erfindung

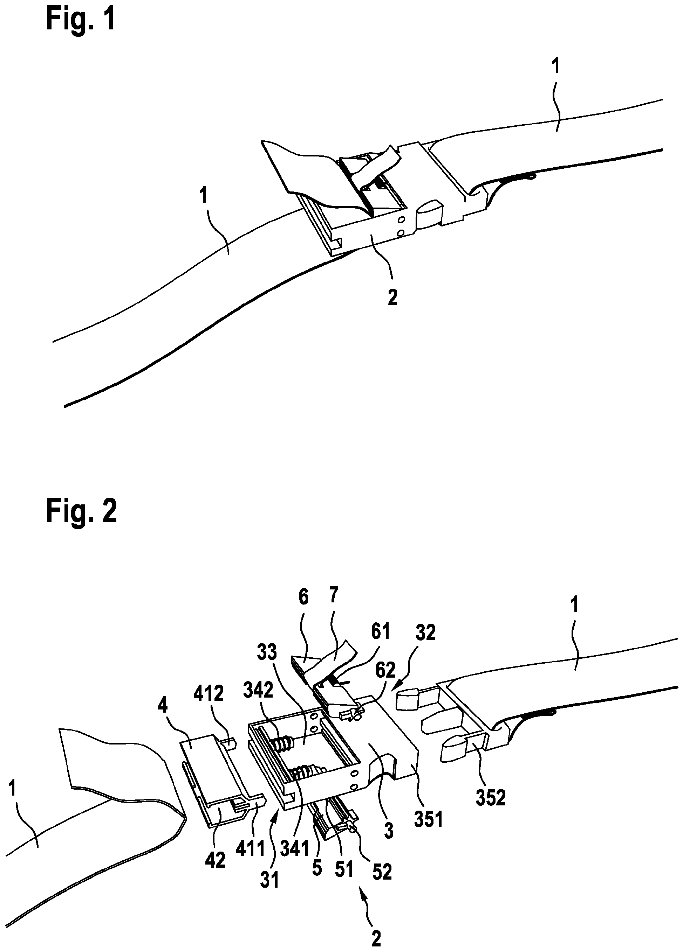

Ein Beckengurt 1 zur Versorgung eines Beckenbruchs mit einer Gurtschnalle 2 gemäß einem Ausführungsbeispiel der Erfindung ist in den Fig. 1 und 2 dargestellt. Die Gurtschnalle 2 weist ein Gehäuse 3 mit einem ersten Ende 31 und einem zweiten Ende 32 auf. Das Gehäuse 3 bildet einen Rahmen um einen Hohlraum 33. Ausgehend vom ersten Ende 31 stehen zwei Federelemente 341, 342 in Form von auf Bolzen geführten Spiralfedern in den Hohlraum 33 hinein. Ein aus zwei Teilen bestehendes Gurtbefestigungselement 351, 352 in Form einer Steckschnalle ist am zweiten Ende 32 des Gehäuses 3 einstückig mit diesem ausgebildet. Ein Schlitten 4 ist in dem Hohlraum 33 beweglich angeordnet. Er weist zwei flache Stifte 411, 412 auf, die in Richtung des zweiten Endes 32 des Gehäuses 3 weisen und an den Seitenwänden des Gehäuses 3 anliegen. Seine dem zweiten Ende 32 zugewandte Außenkontur ist dreieckig konvex und weist eine aufgeraute Oberfläche auf. Ein Hohlraum 42 im Inneren des Schlitten 4 umschließt die Federelemente 341, 342. Zwei Klemmbacken 5, 6 sind jeweils drehbar am zweiten Ende 32 des Gehäuses 3 in dem Hohlraum 33 angeordnet. Jede Klemmbacke 5, 6 weist jeweils ein Schenkelfederelement 51, 61 in Form einer Metallfeder auf, die in das Gehäuse 3 eingreift. Mittels Drehachsen 52, 62 sind die Klemmbacken 5, 6 im Gehäuse 3 drehbar befestigt. Dabei sind die Schenkelfederelemente 51, 61 so angeordnet, dass sie die Klemmbacken 5, 6 in eine geschlossene Position drücken. In dieser geschlossenen Position weisen sie gemeinsam eine in Richtung des Schlittens 4 im Wesentlichen dreieckig konkave Außenkontur auf. An ihrer jeweiligen Außenkontur weist jede Klemmbacke 5, 6 eine aufgeraute Oberfläche auf. An einer der Klemmbacken 6 ist ein Textilband als Bedienelement 7 so angenäht, dass man die Klemmbacke 6 in eine geöffnete Position bewegen kann, wenn man an diesem orthogonal zu der Richtung zieht, in welche die Stifte 411,412 weisen.

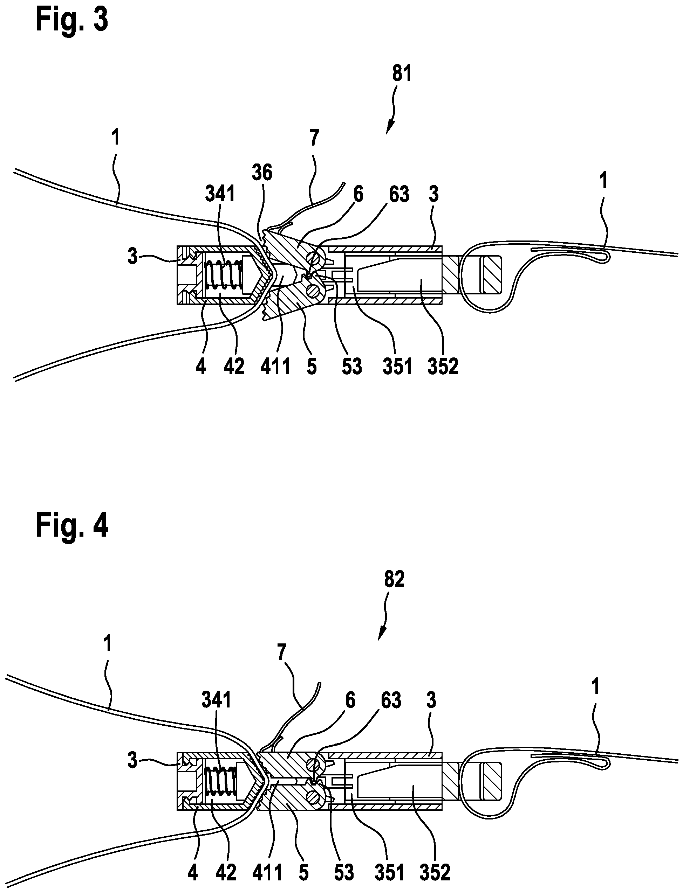

Fig. 3 zeigt eine geöffnete Position 81 der Klemmbacken 5, 6. Die Federelemente 341, 342 befinden sich in einer Ruheposition, in der sie den Schlitten 4 und damit die Stifte 411, 412 in Richtung der Klemmbacken 5, 6 drücken. Die Stifte 411, 412 drücken die Klemmbacken 5, 6 dadurch auseinander. In einem Gurtführungsbereich 36 zwischen den Klemmbacken 5, 6 und dem Schlitten 4 kann der Gurt 1 deshalb angezogen werden. Zwar bewirkt die aufgeraute Oberfläche des Schlittens 4 eine erhöhte Reibung zwischen diesem und dem Gurt 1, dies steht einem Anziehen jedoch nicht entgegen.

Sobald eine mittels der Federelemente 341, 342 voreingestellte Zugkraft von vorliegend 140 N erreicht wurde, wirkt diese der Federkraft der Federelemente 341, 342 so weit entgegen, dass der Schlitten, wie in Fig. 4 dargestellt ist, in Richtung des ersten Endes 31 des Gehäuses 3 bewegt wird. Die Stifte 411, 412 werden dabei so weit in Richtung des ersten Endes 31 zurückgezogen, dass sie die Klemmbacken 5, 6 nicht mehr auseinanderdrücken können. Die Schenkelfederelemente 51, 52 drücken diese nun zusammen. Durch eine Verzahnung 53, 63 zwischen den Klemmbacken 5, 6 erfolgt die resultierende Schließbewegung der Klemmbacken 5, 6 symmetrisch. Hierdurch wird eine geschlossene Position 82 erreicht, in welcher der Gurt 1 im Gurtführungsbereich 36 zwischen der Außenkontur des Schlittens 4 und der nun in geschlossenem Zustand korrespondierenden Außenkontur der Klemmbacken 5, 6 eingeklemmt wird. Mittels der Federelemente 341, 342 drückt der Schlitten 4 den Gurt 1 dabei fest an die Klemmbacken 5, 6 an, Zusammen mit der aufgerauten Außenkontur des Schlittens 4 und der Klemmbacken 5, 6 verhindert dies ein weiteres Anziehen des Gurtes 1.

Ein Lockern des Gurtes 1 kann erfolgen, indem an dem Bedienelement 7 gezogen wird. Hierdurch werden die Klemmbacken 5, 6 aufgrund ihrer Verzahnung 53, 63 symmetrisch geöffnet und die Federelemente 341, 342 können, sofern keine externe Zugkraft mehr auf den Gurt 1 wirkt, in ihrer Ruheposition zurückkehren.

## Patentansprüche

1. Gurtschnalle (2) für einen Beckengurt (1) zur Versorgung eines Beckenbruchs, aufweisend
- ein Gehäuse (3) mit einem ersten Ende (31) und einem zweiten Ende (32),
- ein Schlitten (4), der in dem Gehäuse (3) zwischen dem ersten Ende (31) und dem zweiten Ende (32) beweglich angeordnet ist,
- mindestens ein Federelement (341, 342), das eingerichtet ist, um den Schlitten (4) in Richtung des zweiten Endes (32) zu drücken,
- mindestens ein Stift (411, 412), der so an dem Schlitten (4) angeordnet ist, dass er in Richtung des zweiten Endes (32) weist, **dadurch gekennzeichnet, dass** die Gurtschnalle weiter aufweist
- zwei Klemmbacken (5, 6), die an dem zweiten Ende (32) des Gehäuses (3) drehbar angeordnet sind, und die Schenkelfederelemente (51, 52) aufweisen, welche sie in eine geschlossene Position (82) drücken,
- einen Gurtführungsbereich (36), der zwischen dem Schlitten (4) und den Klemmbacken (5, 6) verläuft,
wobei der mindestens eine Stift (411, 412), wenn sich das mindestens eine Federelement (341, 342) in einer Ruheposition befindet, die Klemmbacken (5, 6) in eine geöffnete Position (81) drückt, und der mindestens eine Stift (411, 412), wenn sich das mindestens eine Federelement (341, 342) in einer komprimierten Position befindet, die Klemmbacken (5, 6) nicht in die geöffnete Position (81) drückt und diese von den Schenkelfederelementen (51, 52) in die geschlossene Position (82) gedrückt werden.

2. Gurtschnalle (2) nach Anspruch 1, **dadurch gekennzeichnet, dass** das mindestens eine Federelement (341, 342) so ausgeführt ist, dass die Klemmbacken (5, 6) in die geschlossene Position (82) gedrückt werden, wenn auf das mindestens eine Federelement (341, 342) eine vorgegebene Kompressionskraft wirkt, die im Bereich von 120 N bis 180 N liegt

3. Gurtschnalle (2) nach Anspruch 1 oder 2, **dadurch gekennzeichnet, dass** der Schlitten (4) zwei Stifte (411, 412) aufweist, die so angeordnet sind, dass der Gurtführungsbereich (36) zwischen den beiden Stiften (411, 412) verläuft.

4. Gurtschnalle (2) nach einem der Ansprüche 1 bis 3, **dadurch gekennzeichnet, dass** der Schlitten (4) einen Hohlraum (42) aufweist, in dem das mindestens ein Federelement (341, 342) angeordnet ist.

5. Gurtschnalle (2) nach einem der Ansprüche 1 bis 4, **dadurch gekennzeichnet, dass** der Schlitten (4) auf seiner den Klemmbacken (5, 6) zugewandten Seite eine Außenkontur aufweist, welche im Wesentlichen komplementär zur Außenkontur der geschlossenen Klemmbacken (5, 6) auf ihrer dem Schlitten (4) zugewandten Seite ist.

6. Gurtschnalle (2) nach einem der Ansprüche 1 bis 5, **dadurch gekennzeichnet, dass** die Klemmbacken (5, 6) miteinander verzahnt sind (53, 63).

7. Gurtschnalle (2) nach einem der Ansprüche 1 bis 6, **dadurch gekennzeichnet, dass** mindestens eine der Klemmbacken (6) ein von außerhalb des Gehäuses (3) zugängliches Bedienelement (7) aufweist, das eingerichtet ist, um die Klemmbacke (6) aus einer geschlossenen (82) in eine geöffnete Position (81) zu bewegen.

8. Gurtschnalle (2) nach einem der Ansprüche 1 bis 7, **dadurch gekennzeichnet, dass** sie am zweiten Ende (32) des Gehäuses (3) ein Gurtbefestigungselement (351, 352) aufweist.

9. Beckengurt (1) zur Versorgung eines Beckenbruchs, der an seinem einen Ende an dem Gurtbefestigungseiement (351, 352) einer Gurtschnalle (2) nach Anspruch 8 befestigt ist und mit seinem anderen Ende durch den Gurtführungsbereich (36) der Gurtschnalle (2) geführt ist.

## Claims

1. Belt buckle (2) for a pelvis belt (1) for supporting a pelvic fracture, having
- a housing (3) having a first end (31) and a second end (32),
- a carriage (4) that is arranged moveably in the housing (3) between the first end (31) and the second end (32),
- at least one spring element (341, 342) that is set up to push the carriage (4) in the direction of the second end (32),
- at least one pin (411, 412) that is arranged on the carriage (4) in such a way that it points in the direction of the second end (32),
**characterised in that** the belt buckle further has
- two clamping jaws (5, 6) that are rotatably arranged on the second end (32) of the housing (3) and which have leg spring elements (51, 52), which they press into a closed position (82),
- a belt guiding region (36), which runs between the carriage (4) and the clamping jaws (5, 6),
wherein, when the at least one spring element (341, 342) is in a rest position, the at least one pin (411, 412) presses the clamping jaws (5, 6) into an open position (81), and, when the at least one spring element (341, 342) is in a compressed position, the at least one pin (411, 412) does not press the clamping jaws (5, 6) into the open position (81), and these are pressed into the closed position (82) by the leg spring elements (51, 52).

2. Belt buckle (2) according to claim 1, **characterised in that** the at least one spring element (341, 342) is designed in such a way that the clamping jaws (5, 6) are pressed into the closed position (82) when a predetermined compression force acts on the at least one spring element (341, 342), said force ranging from 120 N to 180 N.

3. Belt buckle (2) according to claim 1 or 2, **characterised in that** the carriage (4) has two pins (411, 412), which are arranged in such a way that the belt guiding region (36) runs between the two pins (411, 412).

4. Belt buckle (2) according to one of claims 1 to 3, **characterised in that** the carriage (4) has a cavity (42) in which the at least one spring element (341, 342) is arranged.

5. Belt buckle (2) according to one of claims 1 to 4, **characterised in that**, on its side facing towards the clamping jaws (5, 6), the carriage (4) has an outer contour which is substantially complementary to the outer contour of the closed clamping jaws (5, 6) on their side facing towards the carriage (4).

6. Belt buckle (2) according to one of claims 1 to 5, **characterised in that** the clamping jaws (5, 6) are interlocked with one another (53, 63).

7. Belt buckle (2) according to one of claims 1 to 6, **characterised in that** at least one of the clamping jaws (6) has an operating element (7) accessible from outside the housing (3), said operating element being set up to move the clamping jaw (6) from a closed (82) into an open position (81).

8. Belt buckle (2) according to one of claims 1 to 7, **characterised in that** it has a belt fixing element (351, 352) on the second end (32) of the housing (3).

9. Pelvis belt (1) for supporting a pelvic fracture, which, on its one end, is fixed on the belt fixing element (351, 352) of a belt buckle (2) according to claim 8 and, with its other end, is guided through the belt guiding region (36) of the belt buckle (2).

## Revendications

1. Boucle de ceinture (2) destinée à une ceinture de pelvis (1) servant à la prise en charge d'une fracture du pelvis, présentant :
- un logement (3) comportant une première extrémité (31) et une deuxième extrémité (32),
- un patin (4) disposé mobile dans le logement (3) entre la première extrémité (31) et la deuxième extrémité (32),
- au moins un élément de ressort (341, 342) conçu pour pousser le patin (4) vers la deuxième extrémité (32),
- au moins une tige (411, 412) agencée sur le patin (4) de manière à pointer vers la deuxième extrémité (32),
**caractérisée en ce que** la boucle de ceinture présente en outre
- deux mâchoires de serrage (5, 6) qui sont disposées, avec faculté de rotation, sur la deuxième extrémité (32) du logement (3) et présentent des éléments de ressort à languette (51, 52) qui les poussent dans une position fermée (82), et
- une zone de passage de ceinture (36) située entre le patin (4) et les mâchoires de serrage (5, 6),
ladite au moins une tige (411, 412) poussant les mâchoires de serrage (5, 6) vers une position ouverte (81) lorsque l'au moins un élément de ressort (341, 342) se trouve en position de repos, et ladite au moins une tige (411, 412) ne poussant pas les mâchoires de serrage (5, 6) vers la position ouverte (81) lorsque ledit au moins un élément de ressort (341, 342) se trouve en position comprimée, celles-ci étant poussées vers la position fermée (82) par les éléments de ressort à languette (51, 52).

2. Boucle de ceinture (2) selon la revendication 1, **caractérisée en ce que** l'au moins un élément de ressort (341, 342) est conçu de manière que les mâchoires de serrage (5, 6) sont poussées dans la position fermée (82) lorsqu'une force de compression prédéterminée, de l'ordre de 120 N à 180 N, agit sur l'au moins un élément de ressort (341, 342).

3. Boucle de ceinture (2) selon la revendication 1 ou 2, **caractérisée en ce que** le patin (4) présente deux tiges (411, 412) disposées de manière que la zone de passage de ceinture (36) se trouve entre les deux tiges (411, 412).

4. Boucle de ceinture (2) selon l'une des revendications 1 à 3, **caractérisée en ce que** le patin (4) présente une cavité (42) dans laquelle est disposé l'au moins un élément de ressort (341, 342).

5. Boucle de ceinture (2) selon l'une des revendications 1 à 4, **caractérisée en ce que** le patin (4) présente, sur son côté tourné vers les mâchoires de serrage (5, 6), un contour extérieur essentiellement complémentaire du contour extérieur des mâchoires de serrage (5, 6) fermées, sur leur côté tourné vers le patin (4).

6. Boucle de ceinture (2) selon l'une des revendications 1 à 5, **caractérisée en ce que** les mâchoires de serrage (5, 6) présentent une denture correspondante (53, 63).

7. Boucle de ceinture (2) selon l'une des revendications 1 à 6, **caractérisée en ce qu'**au moins une des mâchoires de serrage (6) présente un élément d'actionnement (7) accessible depuis l'extérieur du logement (3) et conçu pour faire passer la mâchoire de serrage (6) d'une position fermée (82) à une position ouverte (81).

8. Boucle de ceinture (2) selon l'une des revendications 1 à 7, **caractérisée en ce qu'**elle présente un élément de fixation de ceinture (351, 352) agencé à la deuxième extrémité (32) du logement (3).

9. Ceinture de pelvis (1) servant à la prise en charge d'une fracture du pelvis, ladite ceinture étant, à l'une de ses extrémités, fixée audit élément de fixation de ceinture (351, 352) d'une boucle de ceinture (2) selon la revendication 8 et, par l'autre de ses extrémités, guidée dans la zone de passage de ceinture (36) de ladite boucle de ceinture (2).
